# EUROPEAN PATENT APPLICATION

(11) **EP 2 447 249 A1**
(43) Date of publication of application: **02.05.2012**
(21) Application number: 10791652.0
(22) Date of filing: 28.06.2010
(51) Int. Cl.: C07D 207/48, C07C 237/20, A61K 31/401, A61K 31/4015

(54) **N-PHENYL-1-SULFONYL-2-PYRROLIDINE CARBOXAMIDE LIBRARIES FOR DRUG DISCOVERY**

(30) Priority: 26.06.2009 ES 200901524
(71) Applicant: Institut Univ. de Ciència i Tecnologia, S.A., 08100 Mollet del Vallès Barcelona (ES)
(72) Inventor: CASTELLS BOLIART, Josep, E-08100 Mollet Del Vallès (ES); MIGUEL CENTENO, David Enrique, E-08100 Mollet Del Vallès (ES); PASCUAL GILABERT, Marta, E-08100 Mollet Del Vallès (ES)
(74) Representative: Ponti Sales, Adelaida
(86) International application number: PCT/ES2010/070433
(87) International publication number: WO 2010/149820

(57) **Abstract**

New compounds are continually being sought for the treatment and prevention of disorders. The invention relates to 1-(sulfonyl)-*N*-phenyl-pyrrolidine 2-carboxamides which can be used in the search for, and identification of, new lead compounds that could modulate the functional activity of a biological target.

## Description

### Field of the invention

The field of the invention is medicinal chemistry. The invention relates to 1-(sulfonyl)-*N*-phenylpyrrolidine-2-carboxamides which can be therapeutically active.

### Background of the invention

Novel compounds are continually sought after to treat and prevent diseases and disorders. Pharmaceutical companies interested in developing new active molecules develop and purchase libraries of chemical compounds in order to screen their biological or pharmacological activity against a particular target, aiming at the identification of new industrially useful products. Therefore, there is a market of customer companies for which the acquisition of novel libraries of chemical compounds, not already biologically explored, is a key issue. Therefore, for the companies whose core business is the design and preparation of libraries of chemical compounds, their commercialization has a clear industrial interest.

In the context of the present invention, "library" is applied to a group of compounds which are structurally related by a main base structure (*scaffold*), but which are distinguishable by the changes in the specific substitute groups linked to the base structure.

Although many research groups work to find novel compounds to be used in the treatment of known or novel diseases, the number of active new chemical entities in the market doesn't grow in the same extension. Over the past few years, there has been a progressive reduction in the number of medicines entering the market mainly due to the more stringent regulatory requirements that have raised the bar on safety and efficacy of new drugs.

The libraries of compounds described in this invention are useful for exploring the chemical space, for incrementing the structural diversity of valuable molecules in the pharmaceutical sector and for increasing the elements of structural recognition in order to study their interaction with biological targets of interest in the pharmaceutical or medical chemistry field. For instance, the molecules may be therapeutically useful as anti-inflammatory or anticoagulation agents, among many other applications

The present invention is useful for a systematic synthesis of large libraries of compounds with industrial applicability. The present invention is useful for making libraries and subsequently optimising the compounds considered as relevant according to the target of interest.

Libraries described in this invention are useful for being biologically and pharmaceutically explored, and therefore to contribute in the research and identification of new drug leads able to modulate the functional activity of a biological target, since these molecules are new sources of chemical diversity not explored up to date. The libraries of the present invention can be explored by means of any known method of biological screening. These methods comprise, but are not limited to, receptor affinity assays, ELISA assays, "southern", "western" and "northern blot", and competitive binding assays.

US 7,126,006 B2 (The Scripps Research Institute) describes glycoluryl type molecules as scaffolds in the preparation of combinatorial libraries.

US 6,939,973 B1 (The Scripps Research Institute) describes glycoluryl type molecules as scaffolds in the preparation of combinatorial libraries.

### Description of the invention

The present invention concerns libraries of chemical compounds wherein each member of said library is a compound of formula (I) 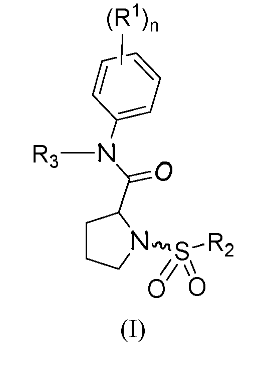 and the salts and stereoisomers thereof, wherein
- **R¹**: is hydrogen, halo, hydroxy, nitro, cyano, carboxyl, C₁₋₆alkyl, C₁₋₆alkoxy, C₁- ₆alkoxyC₁₋₆alkyl, C₁₋₆alkylcarbonyl, amino, mono- or diC₁₋₆alkylamino, azido, mercapto, polyhaloC₁₋₆alkyl, and polyhaloC₁₋₆alkoxy, aryl, Het;
- **R²**: is C₃₋₇cycloalkyl optionally substituted with C₁₋₆alkyl; C₁₋₆alkyl optionally substituted with C₃₋₇cycloalkyl or aryl; C₂₋₆alkenyl optionally substituted with C₃- ₇cycloalkyl or aryl; aryl; Het; or -NR^{4a}R^{4b}, wherein R^{4a} and R^{4b} are, each independently, C₁₋₆alkyl, or R^{4a} and R^{4b} together with the nitrogen to which they are attached form a 5- or 6-membered saturated heterocyclic ring;
- **R³**: is C₁₋₆alkylcarbonyl, C₁₋₆alkyl optionally substituted with aryl, C₁₋₆alkoxyC₁₋₆alkyl, or C₃₋₇cycloalkyl, C₁₋₆alkyl optionally substituted with Het;
- **n**: is one, two, three, four or five;
- each **aryl**: as a group or part of a group is phenyl or naphthalenyl, each optionally substituted with one, two or three substituents selected from halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, and polyhaloC₁₋₆alkoxy;
- each **Het**: as a group or part of a group is a monocyclic ring with 5 or 6 ring atoms or a bicyclic ring structure comprising a 6 membered ring fused to a 4, 5, or 6 membered ring; each of the rings being saturated, partially unsaturated, or completely unsaturated; at least one of the rings containing 1 to 4 heteroatoms each independently selected from nitrogen, oxygen and sulphur; and any one of the rings being optionally substituted with one, two or three substituents each independently selected from the group consisting of halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁- ₆alkylamino, azido, mercapto, polyhaloC₁₋₆alkyl, polyhaloC₁₋₆alkoxy, and C₃- ₇cycloalkyl.

The invention further relates to methods for the preparation of the libraries of compounds where each member of said library is a compound of formula (I), the N-oxides, addition salts, quaternary amines, metal complexes, and stereochemically isomeric forms thereof, their intermediates, and the use of the intermediates in the preparation of the compounds of formula (I).

The invention relates to the libraries of compounds of formula (I) *per se,* the N-oxides, addition salts, quaternary amines, metal complexes, and stereochemically isomeric forms thereof, for use as a drug. In addition, the present invention relates to pharmaceutical compositions including the compounds mentioned above for being administrated to patients for the treatment of inflammation.

The invention also relates to the use of libraries of compounds of formula (I), or N-oxide, addition salt, quaternary amine, metallic complex and stereochemically isomeric forms thereof, for the manufacture of a medicament for the treatment of a disease or conditions such as inflammation or coagulation. Likewise, the present invention relates to the use of the compound of formula (I), or N-oxide, addition salt, quaternary amine, metallic complex and stereochemically isomeric forms thereof, for use in the treatment of a disease or conditions such as inflammation or coagulation. Or the invention relates to a method for treating a disease or conditions such as inflammation or coagulation in a warm-blooded animal, said method comprising administering an effective amount of compound of formula (I), or N-oxide, addition salt, quaternary amine, metallic complex and stereochemically isomeric forms thereof.
From now onwards in the present specification, when using the term "libraries of compounds of formula (I) or "libraries of compounds where each member of the library is a compound of formula (I)" or "the present libraries" or "the present compounds" or similar terms, is meant to include in the libraries of compounds of formula (I), all and each of the subgroups thereof, N-oxides, addition salts, quaternary amines, metallic complex and stereochemically isomeric forms.

The present disclosure also includes the prodrugs of compounds of formula (I).

As used in the foregoing and hereinafter, the following definitions apply unless otherwise noted.

The term halo is generic to fluoro, chloro, bromo and iodo.

The term "polyhaloC₁₋₆alkyl" as a group or part of a group, e.g. in polyhaloC₁₋₆alkoxy, is defined as mono- or polyhalo substituted C₁₋₆alkyl, in particular C₁₋₆alkyl substituted with up to one, two, three, four, five, six, or more halo atoms, such as methyl or ethyl with one or more fluoro atoms, for example, difluoromethyl, trifluoromethyl, trifluoroethyl. Preferred is trifluoromethyl. Also included are perfluoroC₁₋₆alkyl groups, which are C₁₋₆alkyl groups wherein all hydrogen atoms are replaced by fluorine atoms, e.g. pentafluoroethyl. In case more than one halogen atom is attached to an alkyl group within the definition of polyhaloC₁₋₆alkyl, the halogen atoms may be the same or different.

As used herein "C₁₋₄alkyl" as a group or part of a group defines straight or branched chain saturated hydrocarbon radicals having from 1 to 4 carbon atoms such as for example methyl, ethyl, 1-propyl, 2-propyl, 1-butyl, 2-butyl, 2-methyl-1-propyl; "C₁₋₆alkyl" encompasses C₁₋₄alkyl radicals and the higher homologues thereof having 5 or 6 carbon atoms such as, for example, 1-pentyl, 2-pentyl, 3-pentyl, 1-hexyl, 2-hexyl, 2-methyl-1-butyl, 2-methyl-1-pentyl, 2-ethyl-1-butyl, 3-methyl-2-pentyl, and the like. Of interest amongst C₁₋₆alkyl is C₁₋₄alkyl.

The term "C₂₋₆alkenyl" as a group or part of a group defines straight and branched chained hydrocarbon radicals having saturated carbon-carbon bonds and one double bond, and having from 2 to 6 carbon atoms, such as, for example, ethenyl (or vinyl), 1-propenyl, 2-propenyl (or allyl), 1-butenyl, 2-butenyl, 3-butenyl, 2-methyl-2-propenyl, 2-pentenyl, 3-pentenyl, 2-hexenyl, 3-hexenyl, 4-hexenyl, 2-methyl-2-butenyl, 2-methyl-2-pentenyl and the like. Of interest amongst C₂₋₆alkenyl is C₂₋₄alkenyl.

C₃₋₇cycloalkyl is generic to cyclopropyl, cyclobutyl, cyclopentyl, cyclohexyl and cycloheptyl.

C₁₋₆alkoxy means C₁₋₆alkyloxy wherein C₁₋₆alkyl is as defined above.

It should be noted that the radical positions on any molecular moiety used in the definitions may be anywhere on such moiety as long as it is chemically stable.

Radicals used in the definitions of the variables include all possible positional isomers unless otherwise indicated. For instance pyridyl includes 2-pyridyl, 3-pyridyl and 4-pyridyl; pentyl includes 1-pentyl, 2-pentyl and 3-pentyl.

When any variable occurs more than one time in any constituent, each definition is independent.

An embodiment of the present invention relates to libraries of compounds of formula (I) o any subgroup of compounds of formula (I) of the invention, as well as N-oxides, salts, and possible stereochemical forms thereof. Another embodiment relates to libraries of compounds of formula (I) o any subgroup of compounds of formula (I) disclosed herein, as well as salts, and possible stereochemical forms thereof.

The compounds of formula (I) making up the libraries of the present invention may have one or more centers of chirality and may exist as stereochemically isomeric forms. The term "stereochemically isomeric forms" as used herein defines all the possible compounds made up of the same atoms bonded by the same sequence of atoms but having different three-dimensional structures which are not interchangeable, which the compounds of formula (I) may possess.

With reference to the instances where *(R)* or *(S)* is used to designate the absolute configuration of a chiral atom within a substituent, the designation is done taking into consideration the whole compound and not the substituent in isolation.

Unless otherwise mentioned or indicated, the chemical designation of a compound encompasses the mixture of all possible stereochemically isomeric forms, which said compound may possess. Said mixture may contain all diastereomers and/or enantiomers of the basic molecular structure of said compound. All stereochemically isomeric forms of the compounds of the present invention both in pure form or mixed with each other are intended to be embraced within the scope of the present invention.

Pure stereoisomeric forms of the compounds and intermediates as mentioned herein are defined as isomers substantially free of other enantiomeric or diastereomeric forms of the same basic molecular structure of said compounds or intermediates. In particular, the term "stereoisomerically pure" concerns compounds or intermediates having a stereoisomeric excess of at least 80% (i.e. minimum 90% of one isomer and maximum 10% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms "enantiomerically pure" and "diastereomerically pure" should be understood in a similar way, but then having regard to the enantiomeric excess, and the diastereomeric excess, respectively, of the mixture in question.

Pure stereoisomeric forms of the compounds and intermediates of the libraries of the invention can be obtained by the application of art-known procedures. For instance, enantiomers may be separated from each other by the selective crystallization of their diastereomeric salts with optically active acids or bases. Examples thereof are tartaric acid, dibenzoyltartaric acid, ditoluoyltartaric acid and camphosulfonic acid. Alternatively, enantiomers may be separated by chromatographic techniques using chiral stationary phases. Said pure stereochemically isomeric forms may also be derived from the corresponding pure stereochemically isomeric forms of the appropriate starting materials, provided that the reaction occurs stereospecifically. Preferably, if a specific stereoisomer is desired, said compound will be synthesized by stereospecific methods of preparation. These methods will advantageously employ enantiomerically pure starting materials.

The diastereomeric racemates of the compounds of formula (I) can be obtained separately by conventional methods. Appropriate physical separation methods that may advantageously be employed are, for example, selective crystallization and chromatography, e.g. column chromatography.

For some of the compounds of formula (I), their N-oxides, salts, solvates, quaternary amines, or metal complexes, and the intermediates used in the preparation thereof, the absolute stereochemical configuration was not experimentally determined. A person skilled in the art is able to determine the absolute configuration of such compounds using art-known methods such as, for example, X-ray diffraction.

The present invention is also intended to include all isotopes of atoms occurring on the present compounds. Isotopes include those atoms having the same atomic number but different mass numbers. By way of general example and without limitation, isotopes of hydrogen include tritium and deuterium. Isotopes of carbon include C-13 and C-14.

The term "prodrug" as used throughout this text means the pharmacologically acceptable derivatives such as esters, amides, and phosphates, such that the resulting *in vivo* biotransformation product of the derivative is the active drug as defined in the compounds of formula (I). The reference by Goodman and Gilman (The Pharmacological Basis of Therapeutics, 8th ed, McGraw-Hill, Int. Ed. 1992, "Biotransformation of Drugs", p 13-15) describing prodrugs generally is hereby incorporated. Prodrugs preferably have excellent aqueous solubility, increased bioavailability and are readily metabolized into the active inhibitors *in vivo.* Prodrugs of a compound of the present invention may be prepared by modifying functional groups present in the compound in such a way that the modifications are cleaved, either by routine manipulation or *in vivo,* to the parent compound.

Preferred are pharmaceutically acceptable ester prodrugs that are hydrolysable *in vivo* and are derived from those compounds of formula (I) having a hydroxy or a carboxyl group. An *in vivo* hydrolysable ester is an ester, which is hydrolysed in the human or animal body to produce the parent acid or alcohol. Suitable pharmaceutically acceptable esters for carboxy include C₁₋₆alkoxymethyl esters for example methoxymethyl, C₁₋₆alkanoyloxymethyl esters for example pivaloyloxymethyl, phthalidyl esters, C₃₋₈cycloalkoxycarbonyloxyC₁₋₆alkyl esters for example 1-cyclohexylcarbonyloxyethyl; 1,3-dioxolen-2-onylmethyl esters for example 5-methyl-1,3-dioxolen-2-onylmethyl; and C₁₋₆alkoxycarbonyloxyethyl esters for example 1-methoxycarbonyl-oxyethyl which may be formed at any carboxy group in the compounds of this invention.

An *in vivo* hydrolysable ester of a compound of the formula (I) containing a hydroxy group includes inorganic esters such as phosphate esters and α-acyloxyalkyl ethers and related compounds which as a result of the *in vivo* hydrolysis of the ester breakdown to give the parent hydroxy group. Examples of α-acyloxyalkyl ethers include acetoxymethoxy and 2,2-dimethylpropionyloxy-methoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. Examples of substituents on benzoyl include morpholino and piperazino linked from a ring nitrogen atom via a methylene group to the 3- or 4-position of the benzoyl ring.

For therapeutic use, salts of the compounds of formula (I) are those wherein the counter-ion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable acid and base addition salts as mentioned hereinabove are meant to comprise the therapeutically active non-toxic acid and base addition salt forms which the compounds of formula (I) are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids.

Conversely said salt forms can be converted by treatment with an appropriate base into the free base form.

The compounds of formula (I) containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine and the like.

The term addition salt as used hereinabove also comprises the solvates which the compounds of formula (I) as well as the salts thereof, are able to form. Such solvates are for example hydrates, alcoholates and the like.

The term "quaternary amine" as used hereinbefore defines the quaternary ammonium salts which the compounds of formula (I) are able to form by reaction between a basic nitrogen of a compound of formula (I) and an appropriate quaternizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl p-toluenesulfonates. A quaternary amine has a positively charged nitrogen. Pharmaceutically acceptable counterions include chloro, bromo, iodo, trifluoroacetate and acetate. The counterion of choice can be introduced using ion exchange resins.

The N-oxide forms of the present compounds are meant to comprise the compounds of formula (I) wherein one or several nitrogen atoms are oxidized to the so-called N-oxide.

It will be appreciated that the compounds of formula (I) may have metal binding, chelating, complex forming properties and therefore may exist as metal complexes or metal chelates. Such metalated derivatives of the compounds of formula (I) are intended to be included within the scope of the present invention.

Some of the compounds of formula (I) may also exist in their tautomeric form. Such forms although not explicitly indicated in the above formula are intended to be included within the scope of the present invention.

One embodiment of the present invention relates to libraries of compounds of formula (I) or of any subgroup of compounds of formula (I), wherein one or more of the following conditions apply:
- **R¹**: is hydrogen, C₁₋₆alkyl, C₁₋₆alkoxy, C₁₋₆alkoxyC₁₋₆alkyl, C₁₋₆alkylcarbonyl, aryl; Het;
- **R²**: is C₁₋₆alkyl optionally substituted with C₃₋₇cycloalkyl or aryl; C₂₋₆alkenyl optionally substituted with C₃₋₇cycloalkyl or aryl; aryl and Het;
- **R³**: is C₁₋₆alkylcarbonyl, C₁₋₆alkyl optionally substituted with aryl, C₁₋₆alkoxyC₁₋₆alkyl, or C₃₋₇cycloalkyl, C₁₋₆alkyl optionally substituted with Het;
- **n**: is one, two or three;
- each **aryl**: as a group or part of a group is phenyl or naphthalenyl, each optionally substituted with one, two or three substituents selected from halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, and polyhaloC₁₋₆alkoxy;
- each **Het**: as a group or part of a group is a monocyclic ring with 5 or 6 ring atoms or a bicyclic ring structure comprising a 6 membered ring fused to a 4, 5, or 6 membered ring; each of the rings being saturated, partially unsaturated, or completely unsaturated; at least one of the rings containing 1 to 4 heteroatoms each independently selected from nitrogen, oxygen and sulphur; and any one of the rings being optionally substituted with one, two or three substituents each independently selected from the group consisting of halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋ ₆alkylamino, azido, mercapto, polyhaloC₁₋₆alkyl, polyhaloC₁₋₆alkoxy, and C₃₋ ₇cycloalkyl.

One embodiment of the present invention relates to libraries of compounds of formula (I) or of any subgroup of compounds of formula (I), wherein one or more of the following conditions apply:
- **R¹**: is hydrogen;
- **R²**: is C₂₋₆alkenyl optionally substituted with C₃₋₇cycloalkyl or aryl; aryl and Het;
- **R³**: is C₁₋₆alkylcarbonyl, C₁₋₆alkyl optionally substituted with aryl, C₁₋₆alkoxyC₁₋₆alkyl, or C₃₋₇cycloalkyl, C₁₋₆alkyl optionally substituted with Het;
- **n**: is one;
- each **aryl**: as a group or part of a group is phenyl or naphthalenyl, each optionally substituted with one or two substituents selected from halo, amino, mono- or diC₁₋ ₆alkylamino, and polyhaloC₁₋₆alkyl;
- each **Het**: as a group or part of a group is a monocyclic ring with 5 or 6 ring atoms or a bicyclic ring structure comprising a 6 membered ring fused to a 4, 5, or 6 membered ring; each of the rings being saturated, partially unsaturated, or completely unsaturated; at least one of the rings containing 1 to 4 heteroatoms each independently selected from nitrogen, oxygen and sulphur; and any one of the rings being optionally substituted with one or two substituents each independently selected from the group consisting of halo and polyhaloC₁₋₆alkyl.

One embodiment of the present invention relates to libraries of compounds of formula (I) or of any subgroup of compounds of formula (I), wherein one or more of the following conditions apply:
- **R¹**: is hydrogen;
- **R²**: is aryl or Het;
- **R³**: is C₁₋₆alkylcarbonyl, C₁₋₆alkyl optionally substituted with aryl, C₁₋₆alkoxyC₁₋₆alkyl, or C₃₋₇cycloalkyl, C₁₋₆alkyl optionally substituted with Het;
- **n**: is one;
- each **aryl**: as a group or part of a group is phenyl or naphthalenyl, each optionally substituted with two, three, four or five substituents selected from halo, amino, mono- or diC₁₋₆alkylamino, and polyhaloC₁₋₆alkyl;
- each **Het**: as a group or part of a group is a monocyclic ring with 5 or 6 ring atoms or a bicyclic ring structure comprising a 6 membered ring fused to a 4, 5, or 6 membered ring; each of the rings being saturated, partially unsaturated, or completely unsaturated; at least one of the rings containing 1 to 4 heteroatoms each independently selected from nitrogen, oxygen and sulphur; and any one of the rings being being optionally substituted with one or two substituents each independently selected from the group consisting of halo and polyhaloC₁₋₆alkyl.

The libraries of compounds of the present invention may be prepared according to the procedures described hereinafter, which are meant to be applicable for as well the racemates, stereochemically pure intermediates or end products, or any stereoisomeric mixtures. The racemates or stereochemical mixtures may be separated into stereoisomeric forms at any stage of the synthesis procedures.

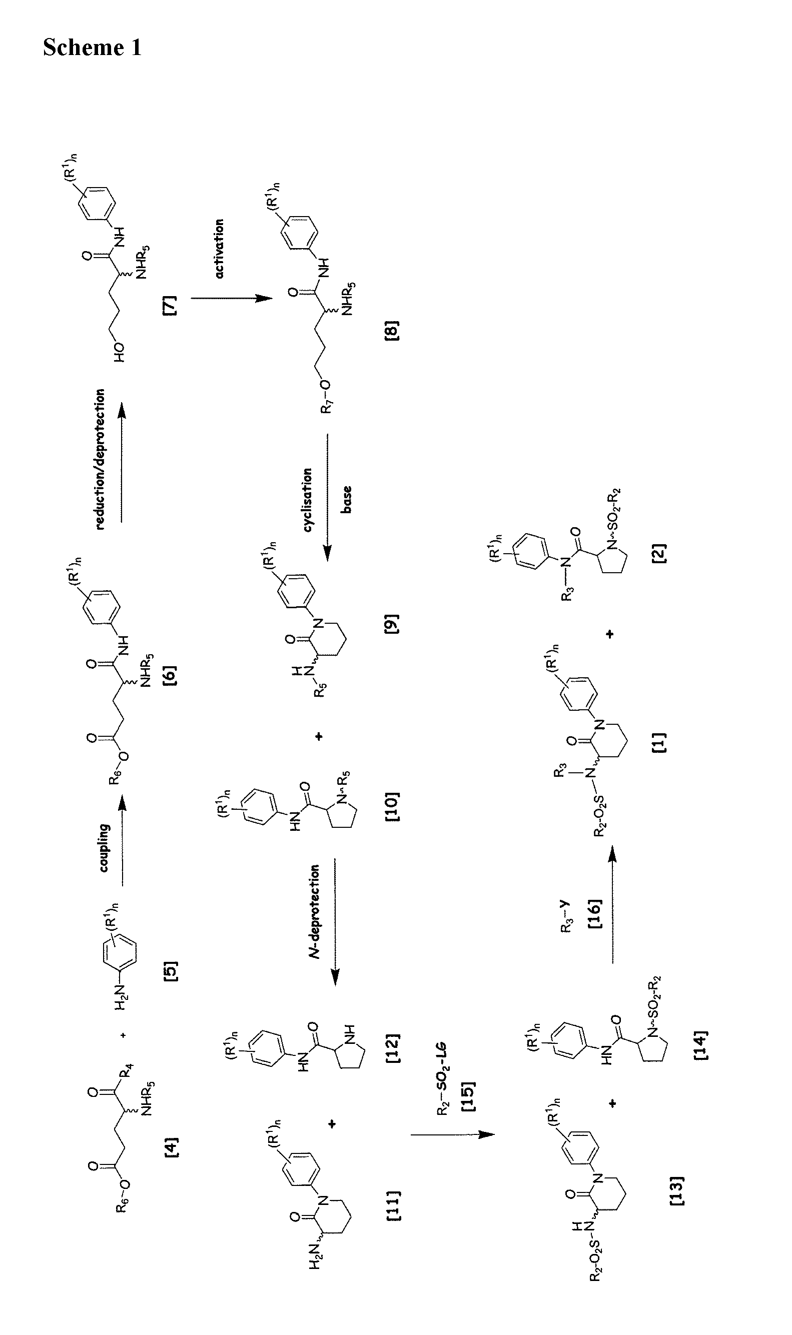

As shown in the above scheme 1, coupling of a compound of formula [4] with the primary amine compound of formula [5] gives the amide derivative compound of formula [6]. The coupling reaction occurs in an organic solvent, such as a chlorinated solvent, preferably dichloromethane, 1,2-dichloroethane or chloroform, at a temperature preferably between -10° C and 40°C, more preferably between 0° C and 25° C. Compound of formula [4] comprises a group -CO-R₄ in the form of an activated carboxyl derivative, such as acid chlorides, anhydrides, or active esters such as *O-*acylisoureas or acyloxyphosphonium derivatives. In a particular embodiment the carbonyl compound is carboxylic acid, the carboxyl activate derivative is *O*-acylisourea and the activating group is a carbodiimide coupling reagent such as dicyclohexylcarbodiimide (DCC), while in another the coupling group is diisopropylcarbodiimide (DIPC).

The corresponding reduction or deprotection reaction of compound [6] yields the alcohol of formula [7]. In a particular embodiment, R₆ group is a benzyl protecting group, and the deprotection reaction comprises the chemoselective reduction of the metal hydride with a reductive agent such as NaBH₄ or Ca(BH₄)₂ in a polar protic solvent, such as ethanol or 2-propanol at a temperature preferably between -10° C and 25°C, more preferably between 0° C and 10° C.

The activation of compound [7] to furnish compound of formula [8] occurs by means of sulfonyl halides, preferably *para*-toluenesulfonyl halides, methanesulfonyl halides or trifluoromethanesulfonyl halides, in the presence of an organic aliphatic or aromatic base, such as pyridine, imidazole, or triethylamine. In a particular embodiment, R₇ group is a methanesulfonyl activating group, and the reaction occurs in a chlorinated solvent, preferably dichloromethane, 1,2-dichloroethane or chloroform, in anhydrous or non anhydrous conditions, at a temperature preferably between -10° C and 40°C, more preferably between 0° C and 25° C.

Treatment of compound [8] under cyclisation conditions yields the lactam compound of formula [9] and the pyrrolidine compound of formula [10]. The reaction occurs in the presence of an inorganic or organic base, such as sodium hydride, potassium *tert-*butoxide or lithium diisopropylamide, at a temperature preferably between -78°C and 60°C, more preferably between -40°C and 0°C. The reaction solvent is a polar aprotic solvent, preferably acetonitrile, tetrahydrofuran, dimethylformamide, or dimethylsulfoxide.

The *N*-deprotection of compounds [9] and [10] yields compounds of formula [11] and [12], respectively where R₅ is an amino protecting group, carbamate, urea-type derivative, amide, cyclic imide, alkyl, aryl, imine, enamine or heteroatom. In a particular embodiment, the protecting group is *tert*-butoxycarbonyl group and the deprotecting agent is trifluoroacetic acid in a chlorinated solvent, preferably dichloromethane, 1,2-dichloroethane or chloroform, at a trifluoroacetic acid composition preferably between 5% and 90%, more preferably between 15% and 70%, at a temperature preferably between 0°C and 45°C, more preferably between 10°C and 30°C.

The substitution reaction of [11] or [12] with compounds of formula R₂-SO₂-LG, where LG means "leaving group", being said LG group preferably an halogen atom, more preferably bromine or chlorine, yields the corresponding substituted sulfonamides of formula [13] and [14], respectively. The reaction solvent is a chlorinated solvent, preferably dichloromethane, 1,2-dichloroethane or chloroform, or a polar aprotic solvent, preferably acetonitrile, tetrahydrofuran, or dimethylformamide, at a temperature preferably between 0° C and 40°C, more preferably between 10° C and 25° C.

Under substitution or coupling conditions with compounds of formula R₃-Y, where Y means "leaving group" in substitution reaction and "activating group" in coupling reactions, being said Y preferably is a halogen atom, more preferably bromine or chlorine in substitution reaction, or an activated carboxyl derivative in coupling reactions, compounds [13] and [14] are converted to the final compounds of formula [1] and [2], respectively. The reaction solvent is anhydrous or non anhydrous polar aprotic solvent, preferably acetonitrile, tetrahydrofuran, or dimethylformamide, at a temperature preferably between -78°C and 60°C, more preferably between -78°C and 25°C.

Both racemic as well as pure enantiomers of [1] and [2] can be accessed by this approach depending on the stereochemical integrity of the starting material. 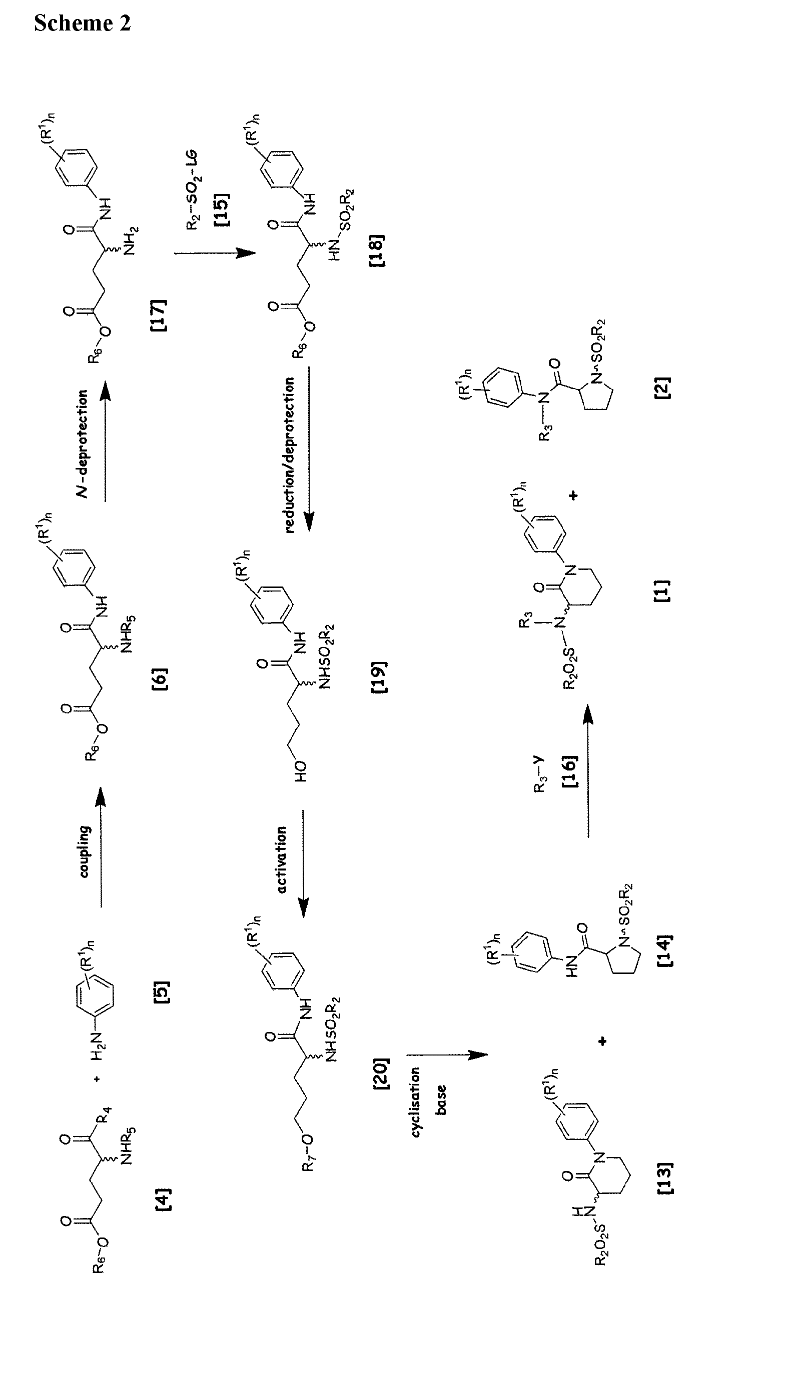 Alternatively, the compounds of formula [1] or [2] can be prepared by the approach as shown in scheme 2. According to scheme 2, coupling of a compound of formula [4] with the compound of formula [5] gives the amide derivative compound of formula [6]. The coupling reaction occurs in an organic solvent, such as a chlorinated solvent, preferably dichloromethane, 1,2-dichloroethane or chloroform, at a temperature preferably between -10° C and 40°C, more preferably between 0° C and 25° C. Compound of formula [4] comprises a group -CO-R₄ in the form of an activated carboxyl derivative, such as acid chlorides, anhydrides, or active esters such as *O-*acylisoureas or acyloxyphosphonium derivatives. In a particular embodiment the carbonyl compound is carboxylic acid, the carboxyl activate derivative is *O*-acylisourea and the activating group is a carbodiimide coupling reagent such as dicyclohexylcarbodiimide (DCC), while in another the coupling group is diisopropylcarbodiimide (DIPC).

The *N*-deprotection of compound [6] yields compounds of formula [17]. In a particular realization the protecting group is *tert*-butoxycarbonyl group and the deprotecting agent is trifluoroacetic acid in a chlorinated solvent, preferably dichloromethane, 1,2-dichloroethane or chloroform, at a trifluoroacetic acid composition preferably between 5% and 90%, more preferably between 15% and 70%, at a temperature preferably between 0°C and 45°C, more preferably between 10°C and 30°C.

The coupling reaction of [17] with compounds of formula R₂-SO₂-LG, where LG means "leaving group", being said LG group preferably an halogen atom, more preferably bromine or chlorine, yields the corresponding substituted sulfonamide of formula [18]. The reaction solvent is a chlorinated solvent, preferably dichloromethane, 1,2-dichloroethane or chloroform, or a polar aprotic solvent, preferably acetonitrile, tetrahydrofuran, or dimethylformamide, at a temperature preferably between 0° C and 40°C, more preferably between 10° C and 25° C.

The corresponding reduction or deprotection reaction of compound [18] yields the alcohol of formula [19]. In a particular embodiment, R₆ group is a benzyl protecting group, and the deprotection reaction comprises the chemoselective reduction of the metal hydride with an reductive agent such as NaBH₄ or Ca(BH₄)₂ in a polar protic solvent, such as ethanol or 2-propanol at a temperature preferably between -10° C and 25°C, more preferably between 0° C and 10° C.

Activation of compound [19] furnishes compound of formula [20]. The reaction occurs by means of sufonyl halides, preferably *para*-toluenesulfonyl halides, methanesulfonyl halides or trifluoromethanesulfonyl halides, in the presence of an organic aliphatic or aromatic base, such as pyridine, imidazole, or triethylamine. The reaction occurs in a chlorinated solvent, preferably dichloromethane, 1,2-dichloroethane or chloroform, in anhydrous or non anhydrous conditions, at a temperature preferably between -10° C and 40°C, more preferably between 0° C and 25° C.

Treatment of compound [20] under cyclisation conditions yields the lactam compound of formula [13] and the pyrrolidine compound of formula [14]. The reaction occurs in the presence of an inorganic or organic base, such as sodium hydride, potassium *tert-*butoxide or lithium diisopropylamide, at a temperature preferably between -78°C and 60°C, more preferably between -40°C and 0°C. The reaction solvent is a polar aprotic solvent, preferably acetonitrile, tetrahydrofuran, dimethylformamide, or dimethylsulfoxide.

Under substitution or coupling conditions with compounds of formula R₃-Y, where Y means "leaving group" in substitution reaction and "activating group" in coupling reactions, being said Y preferably is a halogen atom, more preferably bromine or chlorine in substitution reaction, or an activated carboxyl derivative in coupling reactions, compounds [13] and [14] are converted to the final compounds of formula [1] and [2], respectively. The reaction solvent is a hydrous or anhydrous polar aprotic solvent, preferably acetonitrile, tetrahydrofuran, or dimethylformamide, at a temperature preferably between -78°C and 60°C, more preferably between -78°C and 25°C.

Both racemic as well as pure enantiomers of [1] and [2] can be accessed by this approach depending on the stereochemical integrity of the starting material.

Likewise, an embodiment of the present invention relates to a process for preparing a library of compounds of formula (I) as described herein, said process comprising
a) reacting in a suitable medium compound of formula (II) with a compound of formula (III) 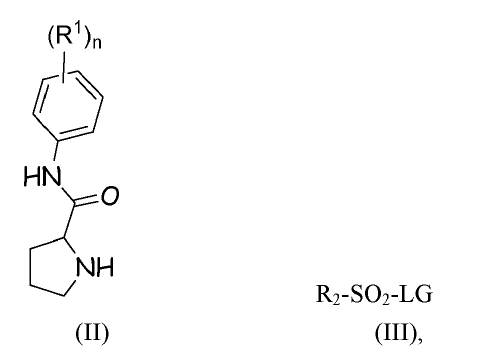 and
b) optionally further reacting in a suitable medium the product of step a) with R₃-Y; wherein
   **R₁, R₂, R₃,** and **n** have the same definition as provided herein;
   **LG** is a leaving group;
   **Y** is an activating group in coupling reactions or a leaving group in substitution reactions.

The suitable medium of the reaction in step a) is a hydrous or anhydrous chlorinated solvent, preferably dichloromethane, 1,2-dichloroethane or chloroform, anhydrous or non anhydrous polar aprotic solvent, preferably acetonitrile, tetrahydrofuran, or dimethylformamide, at a temperature preferably between 0° C and 40°C, more preferably between 0° C and 25° C.

The suitable medium of the reaction in step b) is in the presence of an inorganic or organic base, such as sodium hydride, potassium *tert*-butoxide or lithium diisopropylamide, at a temperature preferably between -78°C and 60°C, more preferably between -78°C and 25°C. The reaction solvent is a polar aprotic solvent, preferably acetonitrile, tetrahydrofuran, dimethylformamide, or dimethylsulfoxide.

The term "leaving group" is preferably a halogen atom, more preferably bromine or chlorine.

The term "activating group" is preferably but not limited to a carboxyl activant in coupling reactions, preferably in the form of an acid chloride, anhydride, or active esters, such as *O*-acylisoureas or acyloxyphosphonium derivatives.

Compounds of formula (I) making up the libraries may be converted into each other following art-known functional group transformation reactions. For example, amino groups may be N-alkylated, nitro groups reduced to amino groups, a halo atom may be exchanged for another halo.

The libraries of compounds of formula (I) may be converted to the corresponding libraries of N-oxide forms following art-known procedures for converting a trivalent nitrogen into its N-oxide form. Said N-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarbo-peroxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. *tert-butyl* hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

Libraries of pure stereochemically isomeric forms of the compounds of formula (I) may be obtained by the application of art-known procedures. Diastereomers may be separated by physical methods such as selective crystallization and chromatographic techniques, e.g., counter-current distribution, liquid chromatography and the like.

The libraries of compounds of formula (I) may be obtained as racemic mixtures of enantiomers which can be separated from one another following art-known resolution procedures. The racemic compounds of formula (I), which are sufficiently basic or acidic may be converted into the corresponding diastereomeric salt forms by reaction with a suitable chiral acid, respectively chiral base. Said diastereomeric salt forms are subsequently separated, for example, by selective or fractional crystallization and the enantiomers are liberated therefrom by alkali or acid. An alternative manner of separating the enantiomeric forms of the compounds of formula (I) involves liquid chromatography, in particular liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound may be synthesized by stereospecific methods of preparation. These methods may advantageously employ enantiomerically pure starting materials.

From a further point of view, the present invention relates to a pharmaceutical composition including a therapeutically effective amount of a compound of formula (I) as disclosed herein, or a compound of any of the subgroups of compounds of formula (I) as disclosed herein, and a pharmaceutically acceptable vehicle. A therapeutically effective amount in this context is an amount enough for stabilizing, reducing or acting prophylactically against a disease or conditions such as inflammation or coagulation. Furthermore, the present invention relates to a process of preparation of a pharmaceutical composition as disclosed herein, including the admixture of a pharmaceutically acceptable vehicle with a therapeutically effective amount of a compound of formula (I), as disclosed herein, or one of the subgroups of compounds of formula (I) as disclosed herein.

Therefore, the compounds of the present invention or any of the subgroups thereof can be formulated in several pharmaceutical forms with the object of being administered. As suitable composition all compositions typically used for the administration of drugs can be mentioned. In order to prepare a pharmaceutical composition of the present invention, a therapeutically effective amount of the compound in particular, optionally in the form of an addition salt or metallic complex, as an active ingredient, is admixed with the pharmaceutically acceptable vehicle, where the vehicle may be present in several forms depending on the intended route of administration. These pharmaceutical compositions are preferred in the form of a single dosage, particularly, for oral, rectal, percutaneous or parenteral injection administration. For example, in the preparation of compositions for oral single dosage forms, any of the pharmaceutical means can be used such as, for example, water, glycols, oils, alcohols, and the like in the case of liquid oral preparations such as suspensions, syrups, mouthwashes, emulsions and solutions; or solid vehicles, such as starch, sugars, kaolin, lubricants, binding agents, disgregating agents and the like in the case of powders, pills, capsules and tablets. Due to its easy administration, tablets and capsules are the most advantageous forms for single dosage, in which case it is obvious that solid pharmaceutical vehicles are used. For parenteral compositions, the vehicle will often include sterile water, at least mostly, although other ingredients should be included, for example, for improving the solubility. For example, injectable solutions can be prepared where the vehicle comprises saline solutions, glucose solutions or a mixture of saline and glucose solutions. Injectable suspensions can also be prepared in which case suitable vehicle liquid, suspending agents and the like can be used. Also included herein are preparations of solid forms which are intended to convert into liquid preparations just before their use. In the suitable compositions for percutaneous administration, the vehicle optionally comprises an enhancing agent for penetration and/or a suitable wetting agent, optionally combined with suitable additives of any kind in minor quantities, additives which are not significantly deleterious for the skin.

It is particularly advantageous to formulate the above pharmaceutical compositions in the form of a single dosage due to the ease of administration and uniformity in the dosage. The form of single dosage, as previously used, relates to discrete physical units suitable as singles dosages, each dosage containing a predetermined quantity of active ingredient calculated for producing the intended therapeutic effect associated with the required pharmaceutical vehicle. Examples of this type of single dosage form are tablets (including scratched or coated tablets), capsules, pills, suppositories, sachets, wafers, injectable solutions or suspensions and the like, and multiple variations thereof.

Accordingly, the compounds of the present invention or any subgroup thereof can be used as a drug. Said use as a drug or method of treatment comprises administering to an individual an effective amount of the compound of formula (I) for combating conditions associated to different diseases, such as inflammation or coagulation, 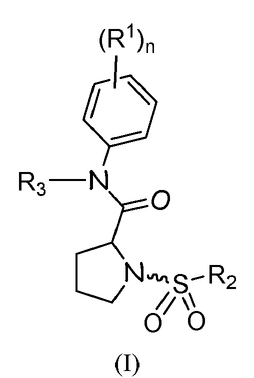 wherein
- **R¹**: is hydrogen, halo, hydroxy, nitro, cyano, carboxyl, C₁₋₆alkyl, C₁₋₆alkoxy, C₁₋ ₆alkoxyC₁₋₆alkyl, C₁₋₆alkylcarbonyl, amino, mono- or diC₁₋₆alkylamino, azido, mercapto, polyhaloC₁₋₆alkyl, and polyhaloC₁₋₆alkoxy, aryl; Het;
- **R²**: is C₃₋₇cycloalkyl optionally substituted with C₁₋₆alkyl; C₁₋₆alkyl optionally substituted with C₃₋₇cycloalkyl or aryl; C₂₋₆alkenyl optionally substituted with C₃₋ ₇cycloalkyl or aryl; aryl; Het; or -NR^{4a}R^{4b}, wherein R^{4a} and R^{4b} are, each independently, C₁₋₆alkyl, or R^{4a} and R^{4b} together with the nitrogen to which they are attached form a 5- or 6-membered saturated heterocyclic ring;
- **R³**: is C₁₋₆alkylcarbonyl, C₁₋₆alkyl optionally substituted with aryl, C₁₋₆alkoxyC₁₋₆alkyl, or C₃₋₇cycloalkyl, C₁₋₆alkyl optionally substituted with Het;
- **n**: is one, two, three, four or five;
- each **aryl**: as a group or part of a group is phenyl or naphthalenyl, each optionally substituted with one, two or three substituents selected from halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, and polyhaloC₁₋₆alkoxy;
- each **Het**: as a group or part of a group is a monocyclic ring with 5 or 6 ring atoms or a bicyclic ring structure comprising a 6 membered ring fused to a 4, 5, or 6 membered ring; each of the rings being saturated, partially unsaturated, or completely unsaturated; at least one of the rings containing 1 to 4 heteroatoms each independently selected from nitrogen, oxygen and sulphur; and any one of the rings being optionally substituted with one, two or three substituents each independently selected from the group consisting of halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋ ₆alkylamino, azido, mercapto, polyhaloC₁₋₆alkyl, polyhaloC₁₋₆alkoxy, and C₃₋ ₇cycloalkyl.

Similarly, the present invention relates to compounds of formula (I) included in the previous paragraph for use in the treatment of diseases or conditions such as inflammation or coagulation.

In addition, the present invention relates to a method of treatment a disease or conditions such as inflammation or coagulation warm-blooded animals, said method including administering an effective amount of compound of formula (I) as indicated in the previous paragraphs, or of a compound from any of the compounds of formula (I).

The term "therapeutically effective amount" as used herein is referred to the amount of a compound or component or active pharmaceutical agent for obtaining a biological or medicinal response in the tissue, system, animal or human being investigated, in the view of the present invention, by an investigator, veterinary, physician or other clinical attendant, including the relief of the symptoms of the disease being treated.

One embodiment of the present invention concerns compounds of formula (IV) or any subgroup of compounds of formula (IV), and the salts and stereoisomers thereof, wherein one or more of the following conditions apply: 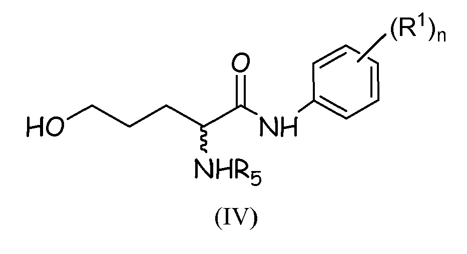 wherein
- **R¹**: is hydrogen, hydroxy, nitro, cyano, carboxyl, C₁₋₆alkyl, C₁₋₆alkoxy, C₁₋₆alkoxyC₁₋ ₆alkyl, C₁₋₆alkylcarbonyl, amino, mono- or diC₁₋₆alkylamino, azido, mercapto, polyhaloC₁₋₆alkyl, and polyhaloC₁₋₆alkoxy, aryl or Het;;
- **R₅**: is an amino protecting group, in the form of carbamate, urea-type derivative, amide, cyclic imide, alkyl, aryl, imine, enamine or heteroatom;
- **n**: is one, two, three, four or five;

The invention further relates to compounds of formula (IV) *per se,* the N-oxides, addition salts, quaternary amines, metal complexes, and stereochemically isomeric forms thereof, for use as synthetic intermediates in the preparation of compounds of formula (I).

One embodiment of the present invention concerns compounds of formula (V) or any subgroup of compounds of formula (V), and the salts and stereoisomers thereof, wherein one or more of the following conditions apply: 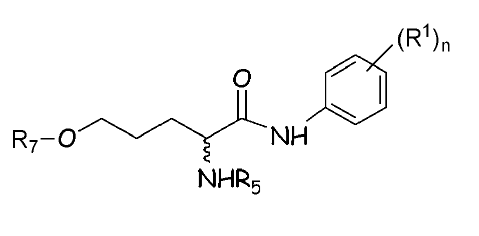 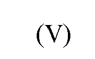 wherein
- **R¹**: is hydrogen, hydroxy, nitro, cyano, carboxyl, C₁₋₆alkyl, C₁₋₆alkoxy, C₁₋₆alkoxyC₁₋ ₆alkyl, C₁₋₆alkylcarbonyl, amino, mono- or diC₁₋₆alkylamino, azido, mercapto, polyhaloC₁₋₆alkyl, and polyhaloC₁₋₆alkoxy, aryl or Het;
- **R₅**: is an amino protecting group, in the form of carbamate, urea-type derivative, amide, cyclic imide, alkyl, aryl, imine, enamine or heteroatom;
- **R₇**: is a hydroxy activating group, preferably in the form of a sulfonate ester, *para-* toluenesulfonyl, methanesulfonyl or trifuloromethanesulfonyl.
- **n**: is one, two, three, four or five.

The invention further relates to compounds of formula (V) *per se,* the N-oxides, addition salts, quaternary amines, metal complexes, and stereochemically isomeric forms thereof, for use as synthetic intermediates in the preparation of compounds of formula (I).

One embodiment of the present invention concerns compounds of formula (VI) or any subgroup of compounds of formula (VI), and the salts and stereoisomers thereof, wherein one or more of the following conditions apply: 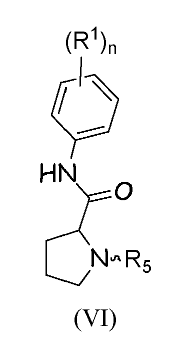 wherein
- **R¹**: is hydrogen, hydroxy, nitro, cyano, carboxyl, C₁₋₆alkyl, C₁₋₆alkoxy, C₁₋₆alkoxyC₁₋ ₆alkyl, C₁₋₆alkylcarbonyl, amino, mono- or diC₁₋₆alkylamino, azido, mercapto, polyhaloC₁₋₆alkyl, and polyhaloC₁₋₆alkoxy, aryl or Het;
- **R₅**: is an amino protecting group, preferably carbamate, urea-type derivative, amide, cyclic imide, alkyl, aryl, imine, enamine or heteroatom.
- n: is one, two, three, four or five.

The invention further relates to compounds of formula (VI) per se, the N-oxides, addition salts, quaternary amines, metal complexes, and stereochemically isomeric forms thereof, for use as synthetic intermediates in the preparation of compounds of formula (I).

### Examples

The following examples are intended to illustrate the present invention and not to limit it thereto.

### Example 1: Preparation of intermediate [6a]: Benzyl 4-(tert-butoxycarbonylamino)-5-oxo-5-(phenylamino) pentanoate

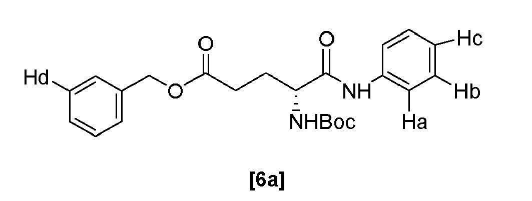

To a stirred solution of Boc-*L*-glutamic acid 5-benzyl ester **[4a]** (41 g, 122 mmol) in anhydrous CH₂Cl₂ (45 ml) at 0°C, was added during 15 minutes a solution of DCC (30.1 g, 146 mmol) in anhydrous CH₂Cl₂ (45 ml). The resulting white solid was sonicated. After that, anhydrous aniline was added dropwise to the reaction mixture over 10 minutes at 0°C (11.1 ml, 122 mmol). The mixture was stirred at room temperature for 40 minutes and filtered through Celite^{®} to remove insoluble material. The resulting liquid was evaporated to dryness and chromatographically purified, yielding the desired product (47.2 g, 94%).
¹H-NMR (400 MHz, CDCl₃): δ: 8.40 (br, 1H, CONHPh), 7.43 (d, 2H, *J*=7.7 Hz, 2Hₐ), 7.28 (d, 2H, *J*=7.7 Hz, H_{b}), 7.20 (m, 5H, 5xH_{d}), 7.02 (t, 1H, *J*=7.4 Hz, H_{c}), 5.35 (d, 1H, *J*=7.8 Hz, CHNHBoc), 5.04 (d, 2H, *J*=2.6 Hz, BnOCH₂), 4.26 (sa, 1H, CH₂CHNHBoc), 2.60-2.52 (mc, 1H, 1xOCOCH₂CH₂), 2.46-2.38 (mc, 1H, 1x OCOCH₂CH₂), 2.21-2.12 (mc, 1H, 1xOCOCH₂CH₂), 1.99-1.90 (mc, 1H, 1x OCOCH₂CH₂), 1.40 (s, 9H, NHCO₂C(CH₃)₃) ppm.
MS: Positive mode [M + Na]⁺ = 435.
MS: Negative mode [M + 2 H₂O - H]⁻ = 447.
CAS nr: [126349-57-3]

### Example 2: Preparation of intermediate [7a]: Tert-butyl 5-hydroxy-l-oxo-(phenylamino)pentan-2-ylcarbamate

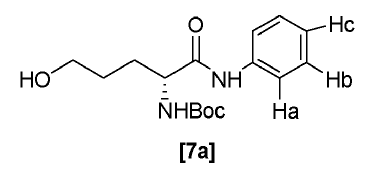

To a stirred suspension of NaBH₄ (12.5g, 342 mmol) in 200 ml EtOH at 0°C was added crushed CaCl₂ (19.9 g, 171 mmol) in portions during 15 min. After that, compound [6a] (35.2 g, 85.8 mmol) was added in portions during 10 minutes. The solution was stirred for 3.5 h, warming to room temperature. The crude was neutralized at 0°C using HCl 0.1 M, and the aqueous phase was extracted in AcOEt. The organic phase was washed using saturated NaCl, dried over anhydrous Na₂SO₄ and evaporated to dryness. The resulting oil residue was chromatographically purified over SiO₂ in Hexane/ AcOEt (40:60), furnishing the desired product (17.4 g, 65%).
¹H-NMR (300 MHz, CDCl₃), δ: 8.85 (br, 1H, CONHPh), 7.50 (dd, 2H, *J*₁=8.7 Hz, *J*₂=1.2 Hz, 2xHₐ), 7.27 (dd, 2H, *J*₁=8.4 Hz, *J*₂=7.8 Hz, 2xH_{b}), 7.08 (t, 1H, *J*₁=7.2 Hz, H_{c}), 5.57 (sa, 1H, *J*=5.7 Hz, CHNHBoc), 4.41 (br, 1H, *J*=5.7 Hz, CHNHBoc), 3.74 (m, 2H, CH₂OH), 2.94 (br, 1H, CH₂OH), 2.0-1.65 (mc, 4H, CH₂CH₂), 1.44 (s, 9H, NHCO₂C(CH₃)₃) ppm.
¹³C-NMR (75 MHz, CDCl₃), δ: 170.6 (CONHPh), 156.2 (C(CH₃)₃), 137.7 (NHCO₂), 128.9 (C_{Ar}-H_{b}), 124.3 (C_{Ar}-H_{c}), 119.9 (C_{Ar}-Hₐ), 62.4 (CH₂OH), 54.6 (CHNHBoc), 30.1 (CH₂CH₂), 28.3 (NHCO₂C(CH₃)₃), 28.0 (CH₂CH₂) ppm.
MS: Positive mode [M+H]⁺ = 309, [M+Na]⁺=331.
MS: Negative mode [M- H]⁻ = 307.

### Example 3: Preparation of intermediate [8a]: 4-(tert-butoxycarbonylamino)-5-oxo-(phenylamino)pentyl methanesulfonate

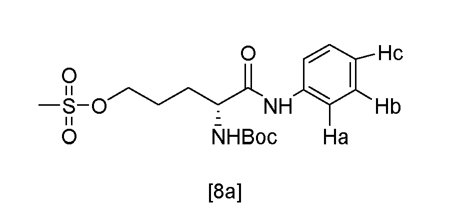

To a stirred solution of compound **[7a]** (0.98 g, 3.19 mmol) in 10 ml anhydrous CH₂Cl₂ was added 0.66 ml of anhydrous Et₃N (4.76 mmol, 1.48 eq) at 0°C. To this solution was added MsCl (3.86 mmol, 1.21 eq) and the mixture was stirred for 2 h at 0°C. After then, the crude was evaporated to dryness, and filtered over SiO₂ using AcOEt as the eluant. Once the filtered was evaporated, finally it was crystallized in acetone at 0°C, yielding 1.12 g (91%) of the desired product.
¹H-NMR (300MHz, CDCl₃): δ 8.435 (s, 1H), 7.512 (dd, *J₁*=7.8 Hz, *J₂*=8.4 Hz, 2H), 7.293 (t, *J*=8.4 Hz, 2H), 7.091 (t, *J*=7.5 Hz, 1H), 5.375 (d, *J*=8.4 Hz, 1H), 4.4 (m, 1H), 4.306 (m, 2H), 3.302 (s, 3H), 2.095-1.750 (m, 4H), 1.446 (s, 9H) ppm.
¹³C-NMR (300MHz, CDCl₃): δ 170.03, 156.15, 137.63, 128.93, 124.41, 119.83, 69.18, 53.67, 37.46, 28.84, 28.28, 25.34 ppm.
MS: Positive mode [M + Na]⁺ = 409.
MS: Negative mode [M+2H₂O - H]⁻ = 421.

### Example 4: Preparation of Intermediate [9a]: tert-butyl-2-oxo-l-phenylpiperidin-3-ylcarbamate

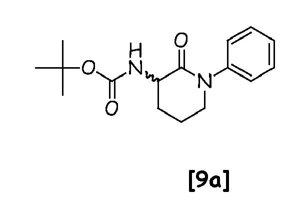

Under inert atmosphere, LDA (1.04 mmol, 2 eq) was added to a solution of compound **[8a]** (0.200 g, 0.52 mmol) in anhydrous THF (5 ml) at 0°C. The solution was stirred for 2.5 h, warming to room temperature. After then, the crude was evaporated to dryness, and purified over Al₂O₃ using Hexane/AcOEt from 70/30 to 50/50 as the eluant, yielding 0.09 g (60%) of the desired product **[9a]** and 0.60 g (40%) of the by-product **[10a].**
¹H-NMR (400MHz, CDCl₃): δ 7.39 (t, *J*=7.6 Hz, 2H_{Ar}), 7.25 (m, 3 H_{Ar}), 5.5 (br, 1H, NHBoc), 4.26 (m, 1H, CHNHBoc), 3.71 (m, 2H, -CHCH₂CH₂CH₂-), 2.61 (m, 1H, CHCH₂CH₂CH₂-), 2.04 (m, 2H, -CHCH₂CH₂CH₂-), 1.71 (m, 1H, CHCH₂CH₂CH₂-), 1.46 (s, 9H, *^{t}*Bu) ppm.
¹³C-NMR (400MHz, CDCl₃): δ 169.94 (CONH), 155.94 (OCONH), 142.47 (C_{q}, C_{Ar}), 129.15 (CH, C_{Ar}), 126.81 (CH, C_{Ar}), 125.64 (CH, C_{Ar}), 79.622 (Cq, *^{t}*Bu), 51.90 (-CHCH₂CH₂CH₂-), 50.14 (-CHCH₂CH₂CH₂-), 28.36 (CH₃, *^{t}*Bu), 27.39 (-CHCH₂CH₂CH₂-), 21.14 ppm (-CHCH₂CH₂CH₂-).
MS: Positive mode [M+H]⁺=291, [M+Na]⁺=313.

### Example 5: Preparation of Intermediate [10a]: Tert-butyl-2-(phenylcarbamoyl)-pyrrolidine-1-carboxylate

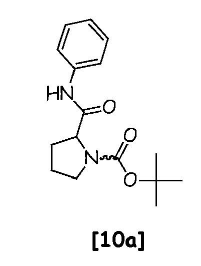

Under inert atmosphere, *^{t}*BuOK (0.070 g, 0.65 mmol) was added to a solution of compound **[8a]** (0.250 g, 0.65 mmol) in anhydrous THF (5.8 ml). The reaction mixture was heated up to 50°C during 1 h. After then, the crude was evaporated to dryness, and purified over SiO₂ using Hexane/AcOEt 50/50 as the eluant, yielding 0.183 g (97%) of the desired product **[10a].**
¹H-NMR (300MHz, CDCl₃): δ 9.5 (br, NHPhe), 7.51 (dd, *J₁*=8.9 Hz, *J₂*=1.2 Hz, 2H_{Ar}), 7.31 (t, *J*=7.8 Hz, 2 H_{Ar}), 7.08 (t, *J*=7.2 Hz, 1 H_{Ar}), 4.4 (br, 1H, CH), 3.4 (br, 2H, CH₂), 1.93 (m, 2H, CH₂), 1.49 (s, 9H, *^{t}*Bu), 1.49 (s, 2H, CH₂) ppm.
MS: Positive mode [M+H]⁺=291, [M+Na]⁺=313.
MS: Negative mode [M - H]⁻=289, [M+2H₂O - H]⁻ = 325.

### Example 6: Preparation of Intermediate [11a]:

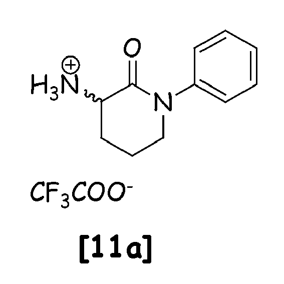

To a stirred solution of **[9a]** (0.08 g, 0.28 mmol) in 1.5 ml of CH₂Cl₂ was added 0.50 ml of trifluoroacetic acid at room temperature, and the mixture was sealed and stirred for 0.5 h. After then, the crude was evaporated to dryness, yielding an orange oily residue of the organic salt **[11a],** which was precipitated using *ⁱ*Pr₂O. The remaining solid was used without further purification.

### Example 7: Preparation of Intermediate [12a]:

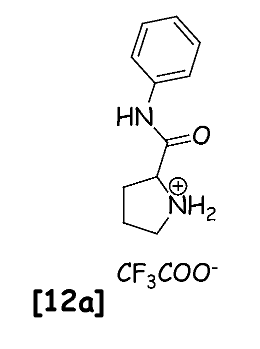

To a stirred solution of **[10a]** (0.90 g, 3.11 mmol) in 13 ml of CH₂Cl₂ was added 5.5 ml of trifluoroacetic acid at room temperature, and the mixture was sealed and stirred for 1 h. After then, the crude was evaporated to dryness, yielding an orange oily residue of the organic salt **[12a],** which was used without further purification.

### Example 8: Preparation of N-phenyl-1-(phenylsulfonyl)pyrrolidine-2-carboxamide:

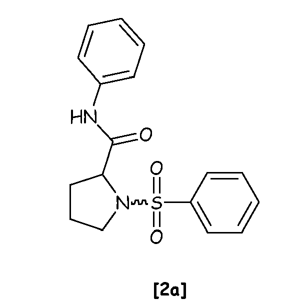

Under inert atmosphere, to a stirred solution of compound [11a] (0.95 g, 3.11 mmol) in 12 ml anhydrous DMF at 0°C was added anhydrous Et₃N (1.50 ml, 10.9 mmol). This mixture was stirred for 5 min, and then PhSO₂Cl (0.6 ml, 4.66 mmol) was added at 0°C.
The reaction was stirred for 2 h at this temperature. Then, the solvent was removed and the crude was chromatographically purified over SiO₂ using Hexane/AcOEt 50/50 as the eluant, yielding 0.93 g (90 %) of the desired product [2].
¹H-NMR (400MHz, CD₃OD): δ 9.70 (br, NHCO), 7.94 (s, 2H, H_{Ar}), 7.70 (s, 1H, H_{Ar}),
7.64-7.58 (4H, H_{Ar}), 7.34 (2H, H_{Ar}), 7.14 (1H, H_{Ar}), 4.26 (1H, -CHCH₂CH₂CH₂-N), 3.63 (1H, 1 x-CHCH₂CH₂CH₂-N), 3.34 (1H, 1 x -CHCH₂CH₂CH₂-N), 2.04 (1H, 1 x - CHCH₂CH₂CH₂-N), 1.95 (2H, 1 x -CHCH₂CH₂CH₂-N + 1 x -CHCH₂CH₂CH₂-N), 1.64 (1H, 1 x -CHCH₂CH₂CH₂-N) ppm.
¹³C-NMR (400MHz, CD₃OD): δ 171.33 (CONH), 137.8 (C_{q}, C_{Ar}), 136.87 (C_{q}, C_{Ar}), 133.09 (CH, C_{Ar}), 129.20 (CH, C_{Ar}), 129.04 (CH, C_{Ar}), 128.54 (CH, C_{Ar}), 128.31 (CH, C_{Ar}), 127.49 (CH, C_{Ar}), 124.47 (CH, C_{Ar}), 120.51 (CH, C_{Ar}), 120.35 (CH, C_{Ar}), 62.68 (CHCH₂CH₂CH₂-N), 49.32 (CHCH₂CH₂CH₂-N), 30.83(CHCH₂CH₂CH₂-N), 24.26 (CHCH₂CH₂CH₂-N) ppm.
MS: Negative mode: [M-H]⁻ = 329

### Example 9: Preparation of 1-(naphthalen-2-ylsulfonyl)-N-phenylpyrrolidine-2-carboxamide

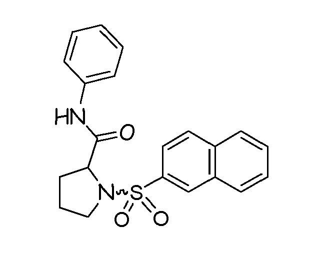

Following a procedure analogous to that described in Example 8, the title compound was obtained in 62 % yield.
MS: Negative mode [M-H]⁻= 379.

### Example 10: Preparation of 1-(5-(dimethylamino)naphthalen-1-ylsulfonyl)-N phenylpyrrolidine-2-carboxamide

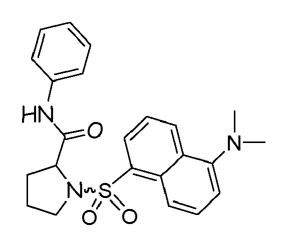

Following a procedure analogous to that described in Example 8, the title compound was obtained in 70% yield.
MS: Negative mode [M-H]⁻= 422.

### Example 11: Preparation of N-(2-(3-carbamoyl-4-hydroxyphenyl)-2-oxoethyl)-N-phenyl-1-(phenylsulfonyl)pyrrolidine-2-carboxamide

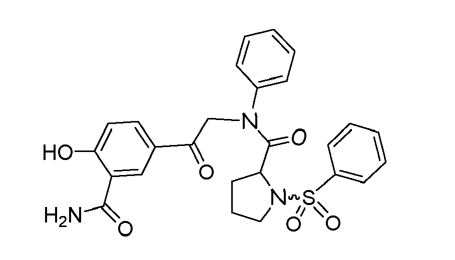

Under inert atmosphere, to a stirred solution of NaH (9 mg, 0.59 mmol) in 0.30 ml anhydrous DMF at 0°C, was added a solution of compound of Example 8 (0.100 g, 0.30 mmol) in 0.60 ml anhydrous DMF. After 1 h at 0°C, a solution of 5-bromoacetyl-2-hydroxybenzamide (0.99 g, 0.36 mmol) in 0.70 ml anhydrous DMF was added to the reaction mixture. This mixture was stirred for 8 h, and then solvent was completely removed. The crude was chromatographically purified over SiO₂ using Hexane/AcOEt 25/75 as the eluant, yielding 0.025 g (16 %) of the desired product.
¹H-NMR (400MHz, CD₃OD): 8.39 (s, 1H, H_{Ar}), 8.11 (sa, 1H, H_{Ar}), 7.94 (s, 2H, H_{Ar}), 7.70 (s, 1H, H_{Ar}), 7.64-7.58 (4H, H_{Ar}), 7.34 (2H, H_{Ar}), 7.14 (1H, H_{Ar}), 7.05 (sa, 1H, H_{Ar}), 4.65 (s, 2H, -CH₂CO-), 4.26 (1H, -CHCH₂CH₂CH₂-N), 3.63 (1H, 1 x-CHCH₂CH₂CH₂-N), 3.34 (1H, 1 x -CHCH₂CH₂CH₂-N), 2.04 (1H, 1 x - CHCH₂CH₂CH₂-N), 1.95 (2H, 1 x -CHCH₂CH₂CH₂-N + 1 x -CHCH₂CH₂CH₂-N), 1.64 (1H, 1 x -CHCH₂CH₂CH₂-N) ppm.
MS: Negative mode: [M-H]⁻ = 506.

## Claims

1. A library of compounds wherein each member of said library is a compound of formula (I) 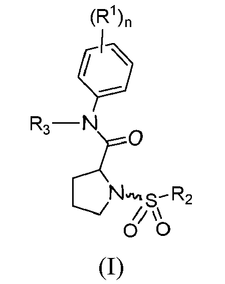 and the salts and stereoisomers thereof, wherein
**R¹** is hydrogen, halo, hydroxy, nitro, cyano, carboxyl, C₁₋₆alkyl, C₁₋₆alkoxy, C₁₋ ₆alkoxyC₁₋₆alkyl, C₁₋₆alkylcarbonyl, amino, mono- or diC₁₋₆alkylamino, azido, mercapto, polyhaloC₁₋₆alkyl, polyhaloC₁₋₆alkoxy, aryl, Het;
**R²** is C₃₋₇cycloalkyl optionally substituted with C₁₋₆alkyl; C₁₋₆alkyl optionally substituted with C₃₋₇cycloalkyl or aryl; C₂₋₆alkenyl optionally substituted with C₃₋ ₇cydoalkyl or aryl; aryl; Het; or -NR^{4a}R^{4b}, wherein R^{4a} and R^{4b} are, each independently, C₁₋₆alkyl, or R^{4a} and R^{4b} together with the nitrogen to which they are attached form a 5- or 6-membered saturated heterocyclic ring;
**R³** is C₁₋₆alkylcarbonyl, C₁₋₆alkyl optionally substituted with aryl, C₁₋₆alkoxyC₁₋₆alkyl, or C₃₋₇cycloalkyl, C₁₋₆alkyl optionally substituted with Het;
n is one, two, three, four or five;
each **aryl** as a group or part of a group is phenyl or naphtyl, each optionally substituted with one, two or three substituents selected from halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, and polyhaloC₁₋ ₆alkoxy;
each **Het** as a group or part of a group is a monocyclic ring with 5 or 6 ring atoms or a bicyclic ring structure comprising a 6 membered ring fused to a 4, 5, or 6 membered ring; each of the rings being saturated, partially unsaturated, or completely unsaturated; at least one of the rings containing 1 to 4 heteroatoms each independently selected from nitrogen, oxygen and sulphur; and any one of the rings being optionally substituted with one, two or three substituents each independently selected from the group consisting of halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋ ₆alkylamino, azido, mercapto, polyhaloC₁₋₆alkyl, polyhaloC₁₋₆alkoxy, and C₃₋ ₇cycloalkyl.

2. A library of compounds according to claim 1, wherein
**R¹** is hydrogen, C₁₋₆alkyl, C₁₋₆alkoxy, C₁₋₆alkoxyC₁₋₆alkyl, C₁₋₆alkylcarbonyl, aryl; Het;
**R²** is C₁₋₆alkyl optionally substituted with C₃₋₇cycloalkyl or aryl or Het; C₂₋₆alkenyl optionally substituted with C₃₋₇cycloalkyl or aryl; aryl and Het;
**R³** or is C₁₋₆alkylcarbonyl, C₁₋₆alkyl optionally substituted with aryl, C₁₋₆alkoxyC₁₋₆alkyl, C₃₋₇cycloalkyl, C₁₋₆alkyl optionally substituted with Het;
**n** is one, two or three;
each **aryl** as a group or part of a group is phenyl or naphthyl, each optionally substituted with one, two or three substituents selected from halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, and polyhaloC₁₋₆alkoxy;
each **Het** as a group or part of a group is a monocyclic ring with 5 or 6 ring atoms or a bicyclic ring structure comprising a 6 membered ring fused to a 4, 5, or 6 membered ring; each of the rings being saturated, partially unsaturated, or completely unsaturated; at least one of the rings containing 1 to 4 heteroatoms each independently selected from nitrogen, oxygen and sulphur; and any one of the rings being optionally substituted with one, two or three substituents each independently selected from the group consisting of halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, polyhaloC₁₋ ₆alkoxy, and C₃₋₇cycloalkyl.

3. A library of compounds according to claims 1-2, wherein
**R¹** is hydrogen, C₁₋₆alkyl, C₁₋₆alkoxy, C₁₋₆alkoxyC₁₋₆alkyl, C₁₋₆alkylcarbonyl, aryl; Het;
**R²** is C₂₋₆alkenyl optionally substituted with C₃₋₇cycloalkyl or aryl; aryl and Het;
**R³** is hydrogen;
**n** is one;
each **aryl** as a group or part of a group is phenyl or naphthyl, each optionally substituted with one or two substituents selected from halo, amino, mono- or diC₁₋ ₆alkylamino, and polyhaloC₁₋₆alkyl;
each **Het** as a group or part of a group is a monocyclic ring with 5 or 6 ring atoms or a bicyclic ring structure comprising a 6 membered ring fused to a 4, 5, or 6 membered ring; each of the rings being saturated, partially unsaturated, or completely unsaturated; at least one of the rings containing 1 to 4 heteroatoms each independently selected from nitrogen, oxygen and sulphur; and any one of the rings being optionally substituted with one or two substituents each independently selected from the group consisting of halo and polyhaloC₁₋₆alkyl.

4. A library of compounds according to claims 1-3, wherein
**R¹** is hydrogen;
**R²** is aryl or Het;
**R³** is C₁₋₆alkylcarbonyl, C₁₋₆alkyl optionally substituted with aryl, C₁₋₆alkoxyC₁₋₆alkyl, or C₃₋₇cycloalkyl, C₁₋₆alkyl optionally substituted with Het;
**n** is one;
each **aryl** as a group or part of a group is phenyl or naphthyl, each optionally substituted with two, three, four or five substituents selected from halo, amino, mono- or diC₁₋₆alkylamino, and polyhaloC₁₋₆alkyl;
each **Het** as a group or part of a group is a monocyclic ring with 5 or 6 ring atoms or a bicyclic ring structure comprising a 6 membered ring fused to a 4, 5, or 6 membered ring; each of the rings being saturated, partially unsaturated, or completely unsaturated; at least one of the rings containing 1 to 4 heteroatoms each independently selected from nitrogen, oxygen and sulphur; and any one of the rings being optionally substituted with one or two substituents each independently selected from the group consisting of halo and polyhaloC₁₋₆alkyl.

5. Pharmaceutical composition comprising a vehicle and as an active ingredient a therapeutic amount of a compound as defined in any of claims 1 to 4.

6. Compound according to any of claims 1 to 5 for use as a medicament.

7. Use of a compound having formula (I) and the salts and stereoisomers thereof, for the manufacture of a medicament for treating a disease or condition 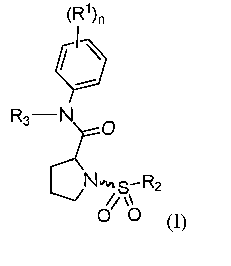 wherein
**R¹** is hydrogen, halo, hydroxy, nitro, cyano, carboxyl, C₁₋₆alkyl, C₁₋₆alkoxy, C₁₋ ₆alkoxyC₁₋₆alkyl, C₁₋₆alkylcarbonyl, amino, mono- or diC₁₋₆alkylamino, azido, mercapto, polyhaloC₁₋₆alkyl, polyhaloC₁₋₆alkoxy, aryl, Het;
**R²** is C₃₋₇cycloalkyl optionally substituted with C₁₋₆alkyl; C₁₋₆alkyl optionally substituted with C₃₋₇cycloalkyl or aryl; C₂₋₆alkenyl optionally substituted with C₃₋ ₇cycloalkyl or aryl; aryl; Het; or -NR^{4a}R^{4b}, wherein R^{4a} and R^{4b} are, each independently, C₁₋₆alkyl, or R^{4a} and R^{4b} together with the nitrogen to which they are attached form a 5- or 6-membered saturated heterocyclic ring;
**R³** is C₁₋₆alkylcarbonyl, C₁₋₆alkyl optionally substituted with aryl, C₁₋₆alkoxyC₁₋₆alkyl, or C₃₋₇cycloalkyl, C₁₋₆alkyl optionally substituted with Het;
**n** is one, two, three, four or five;
each **aryl** as a group or part of a group is phenyl or naphtyl, each optionally substituted with one, two or three substituents selected from halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, and polyhaloC₁₋ ₆alkoxy;
each **Het** as a group or part of a group is a monocyclic ring with 5 or 6 ring atoms or a bicyclic ring structure comprising a 6 membered ring fused to a 4, 5, or 6 membered ring; each of the rings being saturated, partially unsaturated, or completely unsaturated; at least one of the rings containing 1 to 4 heteroatoms each independently selected from nitrogen, oxygen and sulphur; and any one of the rings being optionally substituted with one, two or three substituents each independently selected from the group consisting of halo, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, polyhaloC₁₋₆alkoxy, and C₃₋₇cycloalkyl.

8. Compound according to claim 7 for use in the treatment of diseases related to inflammation or conditions.

9. Method of treatment of a disease or condition in a warm-blooded animal, said method comprising administering an effective amount of a compound according to claim 7.

10. A process for preparing a library of compounds wherein each member of said library is a compound as claimed in any one of claims 1-9, said process comprising
a) reacting in a suitable medium compound of formula (II) with a compound of formula (III) 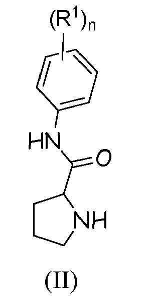
R₂-SO₂-LG (III),
and
b) optionally, further reacting in a suitable medium the product of step a) with R₃-Y; wherein
**R₁, R₂, R₃,** and **n** have the same definition as provided in any one of claims 1-7; LG is a leaving group; preferably a halogen atom, more preferably bromine or chlorine,
**Y** is an activating group in coupling reactions or a leaving group in substitution reactions,
wherein, in substitution reactions Y is preferably a halogen atom, more preferably bromine or chloride;
wherein in coupling reactions Y is preferably an carboxyl derivative activating agent, preferably acid chlorides, anhydride group, or an active ester, preferably *O-*acylisoureas or acyloxyphosphonium derivatives,
wherein the suitable medium of the reaction in step a) is a non-anhydrous or anhydrous chlorinated solvent, preferably dichloromethane, 1,2-dichloroethane or chloroform, or an anhydrous or non anhydrous polar aprotic solvent, preferably acetonitrile, tetrahydrofuran, or dimethylformamide, at a temperature preferably between 0° C and 40°C, more preferably between 0° C and 25° C,
wherein the suitable medium of the reaction in step b) is in the presence of an inorganic or organic base, preferably sodium hydride, potassium *tert*-butoxide or lithium diisopropylamide, at a temperature preferably between -78°C and 60°C, more preferably between -78°C and 25°C,
and wherein the reaction solvent is a polar aprotic solvent, preferably acetonitrile, tetrahydrofuran, dimethylformamide, or dimethylsulfoxide.

11. A compound of formula (IV) 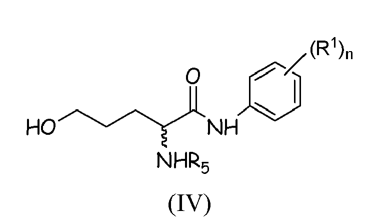 and the salts and stereoisomers thereof, wherein
**R¹** is hydrogen, hydroxy, nitro, cyano, carboxyl, C₁₋₆alkyl, C₁₋₆alkoxy, C₁₋₆alkoxyC₁₋ ₆alkyl, C₁₋₆alkylcarbonyl, amino, mono- or diC₁₋₆alkylamino, azido, mercapto, polyhaloC₁₋₆alkyl, and polyhaloC₁₋₆alkoxy, aryl or Het;
**R₅** is an amino protecting group, preferably carbamate, urea derivative, amide, cyclic imide, alkyl, aryl, imine, enamine or heteroatom;
**n** is one, two, three, four or five.

12. Use of a compound of formula (IV), the N-oxides, addition salts, quaternary amines, metal complexes, and stereochemically isomeric forms thereof, as intermediate in the preparation of a library of compounds wherein each member of said library is a compound formula (I).

13. Use of a library of compounds wherein each member of said library is a compound formula (IV), the N-oxides, addition salts, quaternary amines, metal complexes, and stereochemically isomeric forms thereof, for being biologically and pharmacologically explored in the search and identification of lead drugs in a drug discovering process.

14. A compound of formula (V): 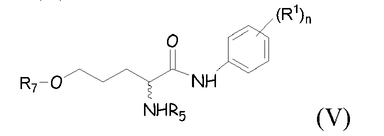 and the salts and stereoisomers thereof, wherein:
**R¹** is hydrogen, hydroxy, nitro, cyano, carboxyl, C₁₋₆alkyl, C₁₋₆alkoxy, C₁₋₆alkoxyC₁₋ ₆alkyl, C₁₋₆alkylcarbonyl, amino, mono- or diC₁₋₆alkylamino, azido, mercapto, polyhaloC₁₋₆alkyl, and polyhaloC₁₋₆alkoxy, aryl, Het;
**R₅** is an amino protecting group, preferably carbamate, urea derivative, amide, cyclic imide, alkyl, aryl, imine, enamine or heteroatom;
**R₇** is a hydroxy activating group, preferably in the form of a sulfonate ester, more preferably *para*-toluenesulfonyl, methanesulfonyl or trifuloromethanesulfonyl.
**n** is one, two, three, four or five.

15. Use of a compound of formula (V), the N-oxides, addition salts, quaternary amines, metal complexes, and stereochemically isomeric forms thereof, as intermediate in the preparation of a library of compounds wherein each member of said library is a compound formula (I).

16. Use of a library of compounds wherein each member of said library is a compound of formula (V), the N-oxides, addition salts, quaternary amines, metal complexes, and stereochemically isomeric forms thereof, for being biologically and pharmaceutically explored in the search and identification of lead drugs in a drug discovering process.

17. A compound of formula (VI) 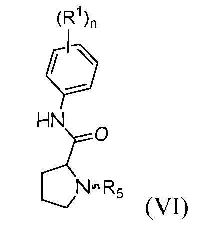 and the salts and stereoisomers thereof, wherein
**R¹** is hydrogen, hydroxy, nitro, cyano, carboxyl, C₁₋₆alkyl, C₁₋₆alkoxy, C₁₋₆alkoxyC₁₋ ₆alkyl, C₁₋₆alkylcarbonyl, amino, mono- or diC₁₋₆alkylamino, azido, mercapto, polyhaloC₁₋₆alkyl, and polyhaloC₁₋₆alkoxy, aryl or Het;
**R₅** is an amino protecting group, preferably carbamate, urea derivative, amide, cyclic imide, alkyl, aryl, imine, enamine or heteroatom.
**n** is one, two, three, four or five.

18. Use of the compounds of formula (VI), the N-oxides, addition salts, quaternary amines, metal complexes, and stereochemically isomeric forms thereof, as intermediate in the preparation of a library of compounds wherein each member of said library is a compound formula (I).

19. Use of a library of compounds wherein each member of said library is a compound formula (VI), the N-oxides, addition salts, quaternary amines, metal complexes, and stereochemically isomeric forms thereof, for being biologically and pharmacologically explored in the search and identification of lead drugs in a drug discovering process.
